# EUROPEAN PATENT APPLICATION

(11) **EP 2 361 568 A1**
(43) Date of publication of application: **31.08.2011**
(21) Application number: 09820640.2
(22) Date of filing: 16.10.2009
(51) Int. Cl.: A61B 17/56, A61B 17/34, A61M 3/00

(54) **BONE CEMENT INJECTION NEEDLE**

(30) Priority: 17.10.2008 JP 2008268681
(71) Applicant: St. Marianna University School of Medicine, Kawasaki-shi Kanagawa 216-8511 (JP); Terumo Kabushiki Kaisha, Tokyo 151-0072 (JP)
(72) Inventor: TAKIZAWA Kenji, Kawasaki-shi Kanagawa 216-8511 (JP); HAYAKAWA Koichi, Ashigarakami-gun Kanagawa 259-0151 (JP); UNO Takuya, Ashigarakami-gun Kanagawa 259-0151 (JP); SARUHASHI Makoto, Ashigarakami-gun Kanagawa 259-0151 (JP)
(74) Representative: TBK
(86) International application number: PCT/JP2009/067913
(87) International publication number: WO 2010/044462

(57) **Abstract**

Disclosed is a bone cement injection needle (100) wherein the bone cement injection needle (100) comprises: a hollow outer needle (110); an outer needle hub (120) affixed to the base end part of the outer needle (110); an inner needle (130) that can be slidably inserted into the hollow part of the outer needle (110); and an inner needle hub (140) affixed to the base end part of the inner needle (130). The outer needle (110) comprises a first side hole (113) located near the tip; a second side hole (114) located near the base end part; and a depressurization passage which connects the first side hole (113) and the second side hole (114).

## Description

### Technical Field

The present invention relates to a puncture needle for injecting bone cement into a bone.

### Background Art

Percutaneous vertebroplasty is a therapeutic method that is used to remove pain due to a compression fracture of a vertebral body of a patient by injecting bone cement into the damaged area of the vertebral body in order to reinforce the vertebral body. Percutaneous vertebroplasty is a relatively new treatment technique, which was first performed in France in 1987, and is now conducted in many medical facilities throughout Japan.

Basically, percutaneous vertebroplasty is based on a transpedicular approach, wherein a hollow puncture needle is inserted into a vertebral body through a pedicle that lies horizontally on the back of the vertebral body, and bone cement is injected into the vertebral body through a passage in the hollow puncture. Generally, a bone biopsy needle is used as the puncture needle for injecting the bone cement. For details, see Japanese Laid-Open Patent Publication No. 2003-024339, for example. The transpedicular approach also includes a two-needle method, in which two needles are inserted respectively into left and right sides of a vertebral body, as well as a single-needle method, in which a needle is inserted into one of the left and right sides of a vertebral body. The single-needle method is considered more preferable because the method is less costly and is less liable to cause complications. The single-needle method requires a smaller dose of radiation, and can be performed in a shorter time than the two-needle method.

### Summary of Invention

However, puncture needles that heretofore have been used are disadvantageous in that when bone cement is injected by a single-needle method, the bone cement is capable possibly of leaking out from the bone.

More specifically, when bone cement is injected by a single-needle method using a conventional puncture needle, the internal pressure in the bone increases as the bone cement is injected, thus forcing the bone cement to leak out from the bone (e.g., into a lumen of a vertebral canal or a vein). Consequently, it has been recommended to perform a two-needle method, which allows the internal pressure of the bone to be reduced using one of the needles, and puts more emphasis on avoiding the problem of internal pressure buildup than on the advantages of the single-needle method, which is preferable for both the patient and the surgeon.

A conventional bone cement injection puncture needle has an outer needle in the form of a single tube. It is desirable to develop a bone cement injection puncture needle having a novel outer needle structure that enables increased functionality.

The present invention has been made in view of the foregoing problems. It is an object of the present invention to provide a bone cement injection puncture needle, which is capable of injecting bone cement into a bone according to a single-needle method without increasing internal pressure inside the bone. Another object of the present invention is to provide a bone cement injection puncture needle with expanded functionality.

The inventors have found that the above problems can be solved by providing a depressurization passage in a needle in addition to a cement injection passage, and have completed the present invention by making further studies.

The present invention is concerned with a bone cement injection puncture needle.

According to the present invention, the bone cement injection puncture needle comprises a hollow outer needle having a cutting edge on a distal end thereof, an outer needle base fixed to a proximal end portion of the outer needle, an inner needle having a needle point on a distal end thereof and slidably inserted in a lumen of the outer needle, and an inner needle base fixed to a proximal end portion of the inner needle, wherein the outer needle includes a first side hole positioned near a distal end portion thereof, a second side hole positioned near a proximal end portion thereof, and a depressurization passage interconnecting the first side hole and the second side hole.

In the above bone cement injection puncture needle, the outer needle includes a hollow outer tube having the first side hole and the second side hole, and a hollow outer needle body rotatably inserted in a lumen of the outer tube and having a groove formed in an outer surface thereof and a sharp cutting edge on a distal end thereof, the inner needle is slidably inserted in a lumen of the outer needle body, and when the outer tube is rotated with respect to the outer needle body, the first side hole and the second side hole are brought into fluid communication with the groove to thereby provide the depressurization passage.

The above bone cement injection puncture needle, further comprises an outer tube base fixed to a proximal end portion of the outer tube.

In the above bone cement injection puncture needle, the outer needle has a length in a range from 10 to 20 cm, and the first side hole and the distal end of the outer needle are spaced from each other by a distance in a range from 0.5 to 2 cm, and the second side hole and the proximal end of the outer needle are spaced from each other by a distance in a range from 0 to 4 cm.

In the above bone cement injection puncture needle, the outer needle has an inside diameter in a range from 1.6 to 3.8 mm.

Since the bone cement injection puncture needle according to the present invention is capable of depressurizing the inside of a bone at the same time that cement is injected into the bone, the bone cement injection puncture needle can inject cement into the bone without allowing the cement to leak from the bone. According to the present invention, therefore, safety and ease of a therapeutic process, such as percutaneous vertebroplasty or artificial bone replacement, can be increased. The depressurization passage according to the present invention is effective not only to actively depressurize the inside of the bone, but also prevents internal pressure buildup in the bone by connecting the inside of the bone to an outer space in order to release pressure from the bone.

According to the present invention, there also is provided a bone cement injection puncture needle.

The bone cement injection puncture needle comprises a hollow outer needle, an outer needle base fixed to a proximal end portion of the outer needle, an inner needle having a needle point on a distal end thereof and slidably inserted in a lumen of the outer needle, and an inner needle base fixed to a proximal end portion of the inner needle, wherein the outer needle includes an inner tube with the inner needle inserted therein, and an outer tube surrounding the inner tube.

According to the present invention, which is arranged as described above, since the outer needle is of a dual-tube structure including the inner tube and the outer tube, certain functions can easily be added to the outer needle because of the dual-tube structure. It is thus possible to provide a depressurization passage between the inner tube and the outer tube, for thereby preventing pressure buildup from developing in the bone upon injection of bone cement into the bone, as is the case with all embodiments of the present invention.

In the above bone cement injection puncture needle, the outer tube includes a first side hole positioned near a distal end portion thereof, and a second side hole positioned near a proximal end portion thereof, the first side hole and the second side hole being held in fluid communication with each other through a depressurization passage formed between the inner tube and the outer tube.

With the above arrangement, when bone cement is injected through a lumen of the outer tube into the bone, gases or liquids (e.g., exudate and blood) in the bone enter from the first side hole into the depressurization passage, and then flow out from the patient's body through the second side hole. Therefore, pressure buildup is prevented from developing in the bone upon injection of bone cement into the bone, so that the bone cement is prevented from leaking out from the bone.

In the bone cement injection puncture needle, the inner tube has a first flaring portion on a proximal end portion thereof, the outer tube has a second flaring portion on a proximal end portion thereof, the first flaring portion is supported by the second flaring portion, and the outer needle base has a tapered support held in abutment against an outer surface of the second flaring portion.

With the above arrangement, since the first flaring portion is supported by the second flaring portion, the inner tube and the outer tube are combined together integrally as the outer needle. Since the second flaring portion is supported by the tapered support of the outer needle base, the outer needle is prevented from being removed from the outer needle base when the puncture needle is pulled out from the bone. When the outer needle is assembled, the first flaring portion and the second flaring portion are superposed on each other, to automatically bring the inner tube into coaxial alignment with the outer tube. Therefore, the inner tube can easily be centered in the outer tube.

In the above bone cement injection puncture, the first flaring portion and the second flaring portion are polygonal in cross section.

With the above arrangement, since the outer needle and the outer needle base are prevented from rotating relatively with respect to each other, when the puncture needle is rotated and pulled from the bone, the outer needle is fixed against rotation with respect to the bone, and the outer needle base cannot rotate with respect to the outer needle. Therefore, the outer needle can easily be pulled out from the bone.

In the bone cement injection puncture needle, the outer tube has a tapered portion on a distal end portion thereof, the tapered portion being progressively tapered toward a tip end thereof, and the inner tube has a distal end portion supported by an inner circumferential surface of the tapered portion.

With the above arrangement, when the outer needle is assembled, the inner tube is inserted into the outer tube until the distal end portion of the inner tube abuts against the tapered portion of the outer tube. Since the inner tube is automatically brought into coaxial alignment with the outer tube, the inner tube can easily be centered in the outer tube. Inasmuch as the distal end portion of the inner tube is supported by the inner circumferential surface of the tapered portion of the outer tube, the inner tube and the outer tube do not need to be joined to each other by a joining means, such as brazing or the like. Therefore, the inner tube and the outer tube can be fabricated easily.

The above bone cement injection puncture needle further comprises an auxiliary connection port fixed to the outer needle and having a passage held in fluid communication with the second side hole.

With the above arrangement, a suction tool or a cleaning liquid injection device can be connected to the auxiliary connection port.

In the bone cement injection puncture needle, the auxiliary connection port is formed integrally with the outer needle base.

With the above arrangement, since the auxiliary connection port and the outer needle base are integral with each other, the number of parts used is small, and the parts can be fabricated easily.

In the above bone cement injection puncture needle, the first side hole comprises a plurality of side holes distributed in circumferential and axial directions of the outer needle.

With the above arrangement, even if liquids within the bone become stuck to some portions of the first side hole, the liquids can flow out into the outer needle from the other first side holes. Consequently, pressure buildup is reliably prevented from developing in the bone.

In the above bone cement injection puncture needle, the outer needle has a foremost end spaced from certain ones of the first side holes, which are positioned most closely to the proximal end of the outer needle by a distance equal to or smaller than 20 mm.

With the above arrangement, the first side holes are appropriately positioned in order to prevent liquids, which flow from within the bone into the outer needle, from leaking into the body of the patient from first side holes that are positioned more closely to the proximal end of the outer needle.

### Brief Description of Drawings

FIG. 1 is an overall view of a bone cement injection puncture needle according to a first embodiment of the present invention;
FIG. 2A is a view of an outer needle of the bone cement injection puncture needle according to the first embodiment of the present invention;
FIG. 2B is a view of an inner needle of the bone cement injection puncture needle according to the first embodiment of the present invention;
FIG. 3A is a view of the bone cement injection puncture needle, with the inner needle inserted therein, according to the first embodiment of the present invention;
FIG. 3B is a view of the bone cement injection puncture needle, with the inner needle removed therefrom, according to the first embodiment of the present invention;
FIG. 4A is a cross-sectional view taken along line IVA-IVA of FIG. 3B;
FIG. 4B is a cross-sectional view taken along line IVB-IVB of FIG. 3B;
FIG. 5 is an overall view of a bone cement injection puncture needle according to a second embodiment of the present invention;
FIG. 6A is a view of an outer needle body of the bone cement injection puncture needle according to the second embodiment of the present invention;
FIG. 6B is a view of an outer tube of the bone cement - injection puncture needle according to the second embodiment of the present invention;
FIG. 6C is a view of an inner needle of the bone cement injection puncture needle according to the second embodiment of the present invention;
FIG. 7A is a cross-sectional view of the bone cement injection puncture needle according to the second embodiment of the present invention, with a first side hole, a second side hole, and a depressurization passage being connected to each other;
FIG. 7B is a cross-sectional view of the bone cement injection puncture needle according to the second embodiment of the present invention, with the first side hole, the second side hole, and the depressurization passage being disconnected from each other;
FIG. 8A is a cross-sectional view taken along line VIIIA-VIIIA of FIG. 7A;
FIG. 8B is a cross-sectional view taken along line VIIIB-VIIIB of FIG. 7B;
FIG. 9 is an overall view of a bone cement injection puncture needle according to a third embodiment of the present invention;
FIG. 10 is an overall view of a bone cement injection puncture needle according to a fourth embodiment of the present invention;
FIG. 11 is a cross-sectional view, partially omitted from illustration, taken along line XI-XI of FIG. 10;
FIG. 12 is a cross-sectional view, partially omitted from illustration, of the bone cement injection puncture needle according to the fourth embodiment of the present invention, with an inner needle removed from an outer needle;
FIG. 13A is a cross-sectional view, partially omitted from illustration, of the bone cement injection puncture needle according to the fourth embodiment of the present invention, with the inner needle being inserted into the outer needle;
FIG. 13B is a cross-sectional view, partially omitted from illustration, of the bone cement injection puncture needle according to the fourth embodiment of the present invention, with an inner needle base mounted on an outer needle base;
FIG. 14 is an enlarged view, partially omitted from illustration, of the bone cement injection puncture needle according to the fourth embodiment of the present invention, showing first side holes formed in the outer needle and regions proximate thereto;
FIG. 15 is an enlarged cross-sectional view, partially omitted from illustration, showing a modified tip end portion of the outer needle of the bone cement injection puncture needle according to the fourth embodiment of the present invention;
FIG. 16 is an enlarged cross-sectional view of a modification of a first flaring portion and a second flaring portion of the bone cement injection puncture needle according to the fourth embodiment of the present invention;
FIG. 17 is an enlarged cross-sectional view of a modification of an inner tube of the bone cement injection puncture needle according to the fourth embodiment of the present invention;
FIG. 18 is a cross-sectional view, partially omitted from illustration, of a bone cement injection puncture needle according to a fifth embodiment of the present invention; and
FIG. 19 is an overall view of a modification of the bone cement injection puncture needle according to the fourth and fifth embodiments of the present invention.

### Description of Embodiments

In the present description, the term "bone cement" refers not only to bone cement (such as a plastic product), but also to bone paste (such as a calcium phosphate product).

According to the present invention, a bone cement injection puncture needle for injecting bone cement into the bone comprises a hollow outer needle having a cutting edge on a distal end thereof, an outer needle base fixed to a proximal end portion of the outer needle, an inner needle having a needle point on a distal end thereof and which is slidably inserted into a lumen of the outer needle, and an inner needle base fixed to a proximal end portion of the inner needle. In the bone cement injection puncture needle, the outer needle includes a first side hole, a second side hole, and a depressurization passage.

The outer needle comprises a metal tube having a bone cement passage formed therein and also having a sharp cutting edge on a distal end thereof. The "sharp cutting edge" is formed by cutting a distal end portion of a metal tube to a predetermined shape, and then polishing the cut surface to a tapered shape. The outer needle may be made of any material which is strong enough to withstand insertion into a bone. For example, the outer needle may be made of stainless steel. The outer needle is not limited to any particular length, but may have a length that is appropriate depending on the purpose of the puncture needle. Often, the length of the outer needle may be in a range from 10 to 20 cm. The outer needle is not limited to any particular inside diameter (diameter of the bone cement passage), but should preferably be of a diameter in a range from 1.6 to 3.8 mm, in view of the viscosity of the bone cement and to facilitate invasion into living tissue.

The bone cement injection puncture needle according to the present invention includes a first side hole, a second side hole, and a depressurization passage, which are provided in the outer needle. The first side hole comprises one or more holes formed in a side wall of the outer needle near a distal end portion thereof, and the second side hole comprises one or more holes formed in a side wall of the outer needle near a proximal end portion thereof. The first side hole and the second side hole are held in fluid communication with the depressurization passage at all times (refer to the first embodiment), or can be brought into fluid communication with the depressurization passage as required (refer to the second embodiment). The depressurization passage comprises a lumen, which is formed in the outer needle separately from the bone cement passage. The quantities of the first side holes and the second side holes are not limited to any particular value, but should preferably be two or more each, in view of the possibility of becoming clogged with bone cement. The number of depressurization passages also is not limited, but may be set to any suitable value depending on the quantities of the first side holes and the second side holes. The quantities of the first side holes, the second side holes, and the depressurization passages may be the same as each other or different from each other.

As described above, the first side hole is formed in the side wall of the outer needle near a distal end portion thereof, and the second side hole is formed in the side wall of the outer needle near a proximal end portion thereof. The positions of the first side hole and the second side hole are established depending on the purpose of the puncture needle, so that when the bone cement injection puncture needle is inserted into a bone in question, the first side hole becomes positioned within the bone, while the second side hole becomes positioned outside the body of the patient. Normally, the tip end of the outer needle and the first side hole are spaced from each other by a distance in a range from about 0.5 to 2 cm, and the proximal end of the outer needle and the second side hole are spaced from each other by a distance in a range from about 0 to 4 cm. The first side hole and the second side hole are not limited to any particular size, and similarly, the depressurization passage is not limited to any particular size (width and depth), insofar as gases and liquids are capable of flowing from the first side hole to the second side hole.

The outer needle may be constituted of one member or of two or more members. For example, the outer needle may comprise a hollow outer tube having a first side hole and a second side hole formed therein, and a hollow outer needle body having a depressurization passage formed therein (refer to the second embodiment). The outer needle body is slidably inserted into a lumen of the outer tube. When the outer tube is rotated with respect to the outer needle body, the first side hole and the second side hole can be opened and closed. The outer tube base may be fixed to a proximal end portion of the outer tube, so that the user (a doctor) can rotate the outer tube easily.

The outer needle base serves as a grip, which is fixed to the proximal end portion of the outer needle. The user (doctor) grips the outer needle base and inserts the outer needle and the inner needle into a bone. The outer needle base is not limited to any particular size or shape, but may be of a size and shape that allows the user (doctor) to grip the outer needle base easily. Normally, the outer needle base includes a bone cement passage formed therein, which is held in fluid communication with the bone cement passage in the outer needle. The bone cement passage has a syringe insertion port in an opening thereof.

As described later, since the distal end of the outer needle and the distal end of the inner needle are combined together into a single needle point, the outer needle and the inner needle should preferably be kept in respective relative positions at least when the bone cement injection puncture needle according to the present invention is inserted into a bone. In order to prevent the inner needle from becoming positionally displaced, the outer needle base should preferably have a fixing mechanism for removably engaging and fixing the inner needle base.

The inner needle comprises a metal bar, which is slidably inserted in the bone cement passage in the outer needle, and having a sharp needle point on a distal end thereof. The inner needle may be made of any material insofar as the material provides sufficient mechanical strength. For example, the inner needle may be made of stainless steel. The inner needle is not limited to any particular outside diameter, but should preferably be of an outside diameter that is substantially the same as the inside diameter of the bone cement passage in the outer needle.

The inner needle is inserted in the bone cement passage in the outer needle, such that the distal end of the inner needle projects from the distal end of the outer needle. The distal end (cutting edge) of the outer needle and the distal end (needle point) of the inner needle are combined together into a single needle point. The needle point, which is formed by the outer needle and the inner needle, is not limited to any particular shape, insofar as the needle point can be inserted into a bone. The needle point may be of a shape selected from among shapes known to those skilled in the art. Examples of shapes of the needle point, which is formed by the outer needle and the inner needle, include a trocar tip, a scoop tip, a side bevel tip, and a diamond tip. The inner needle has a length set to a value such that, when the inner needle is inserted in the bone cement passage in the outer needle, the distal end (cutting edge) of the outer needle and the distal end (needle point) of the inner needle can be combined together into a single needle point. Normally, the length of the inner needle is substantially the same as the sum of the length of the bone cement passage in the outer needle added to the length of the bone cement passage in the outer needle base.

The inner needle base serves as a grip, which is fixed to the proximal end portion of the inner needle. After having inserted the outer needle, along with the inner needle that is inserted therein, into a bone, the user (doctor) grips the inner needle base and removes the inner needle from the outer needle. Then, the user (doctor) installs a syringe, which is filled with bone cement, on an insertion port of the outer needle base. After having injected the bone cement, the user (doctor) grips the inner needle base and inserts the inner needle into the outer needle, thereby pushing the bone cement from the outer needle into the bone. The inner needle base is not limited to any particular size or shape, but may be of a size and shape that allows the user (doctor) to grip the inner needle base easily.

Embodiments of the present invention will be described below with reference to the drawings. However, the scope of the present invention is not necessarily limited to the embodiments.

### [First Embodiment]

According to a first embodiment, a bone cement injection puncture needle includes an outer needle, which comprises a single member.

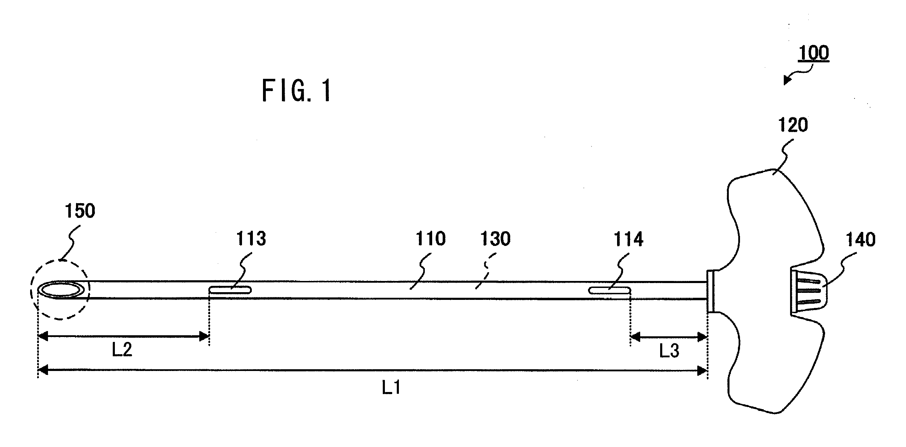

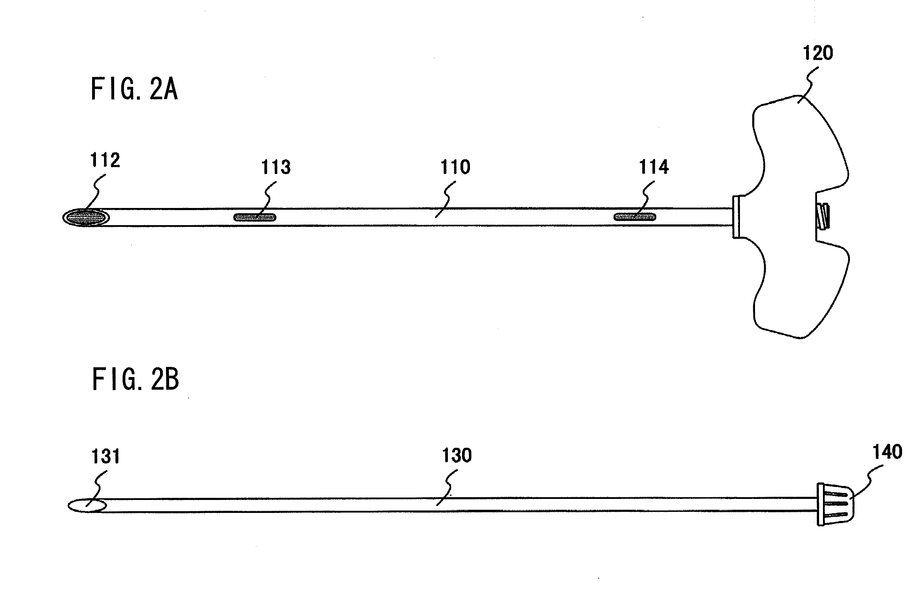

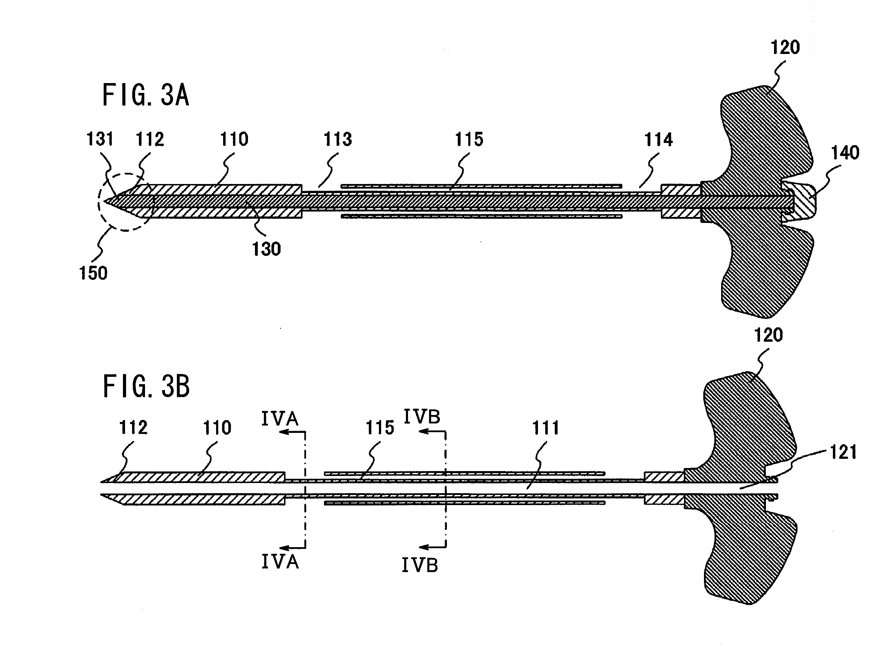

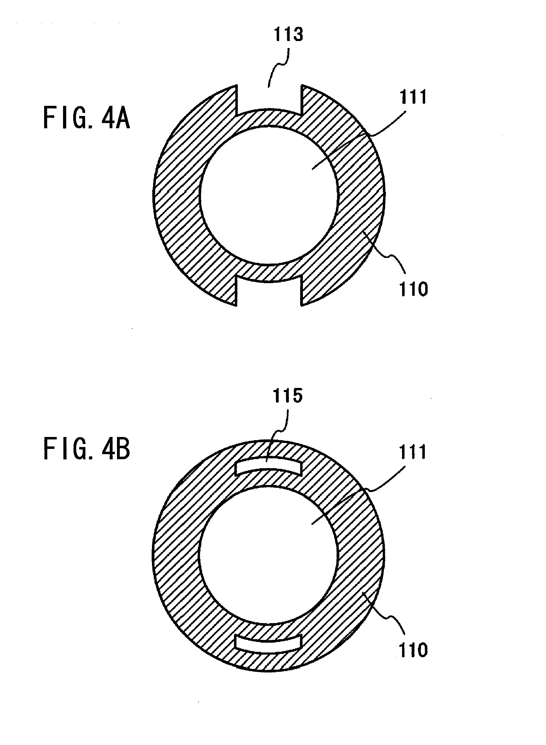

FIGS. 1 to 4 are views showing the structure of the bone cement injection puncture needle according to the first embodiment. FIG. 1 is an overall view of the bone cement injection puncture needle, with an outer needle and an inner needle combined together, FIG. 2A is a view of an outer needle of the bone cement injection puncture needle, FIG. 2B is a view of an inner needle of the bone cement injection puncture needle, FIG. 3A is a view of the bone cement injection puncture needle, with the inner needle and the outer needle combined together, FIG. 3B is a view of the outer needle of the bone cement injection puncture needle, FIG. 4A is a cross-sectional view taken along line IVA-IVA of FIG. 3B, and FIG. 4B is a cross-sectional view taken along line IVB-IVB of FIG. 3B. In order to more clearly show the internal structure thereof, the ratio of the length and thickness of the needle in the cross-sectional views of FIG. 3 is different from the ratio shown in FIGS. 1 and 2.

As shown in FIGS. 1 to 4, the bone cement injection puncture needle 100 according to the first embodiment comprises an outer needle 110, an outer needle base 120, an inner needle 130, and an inner needle base 140.

The outer needle 110 comprises a metal tube (e.g., a stainless steel tube) having a bone cement passage 111 formed therein and a sharp cutting edge 112 on a distal end thereof. The outer needle 110 has a length (indicated by "L1" in FIG. 1) in a range from about 10 to 20 cm, and an inside diameter in a range from about 1.8 to 2.4 mm.

The outer needle 110 also has first side holes 113, second side holes 114, and depressurization passages 115 (see FIG. 3) formed therein. The first side holes 113 are formed in a side wall of the outer needle 110 near the distal end portion thereof, and are held in fluid communication with the depressurization passages 115. Similarly, the second side holes 114 are formed in a side wall of the outer needle 110 near the proximal end portion thereof, and are held in fluid communication with the depressurization passages 115. The depressurization passages 115 comprise lumens formed in the outer needle 110 separately from the bone cement passage 111. The depressurization passages 115 interconnect the first side holes 113 and the second side holes 114 (see FIGS. 3 and 4). With the bone cement injection puncture needle 100 according to the first embodiment, the single outer needle 110 has two first side holes 113, two second side holes 114, and two depressurization passages 115 (see FIGS. 3 and 4).

As described above, the first side holes 113 are formed in a side wall of the outer needle 110 near the distal end portion thereof, and the second side holes 114 are formed in a side wall of the outer needle 110 near the proximal end portion thereof. The distal end portion of the outer needle 110 is spaced from the first side holes 113 by a distance (indicated by "L2" in FIG. 1) in a range from about 0.5 to 2 cm. The proximal end portion of the outer needle 110 is spaced from the second side holes 114 by a distance (indicated by "L3" in FIG. 1) in a range from about 0 to 4 cm. The first side holes 113 and the second side holes 114 are not limited to any particular size. Also, the depressurization passages 115 are not limited to any particular diameter, insofar as gases and liquids can flow from the first side holes 113 and into the second side holes 114.

The outer needle base 120 comprises a resin-molded member (grip) bonded to the proximal end portion of the outer needle 110 (see FIG. 2A). The outer needle base 120 has a bone cement passage 121 formed therein, which is held in fluid communication with the bone cement passage 111 (see FIG. 3B). The bone cement passage 121 has an opening, which functions as an insertion port for enabling a syringe to be inserted therein. The outer needle base 120 also has an externally threaded surface to which the inner needle base 140 is removably fastened (see FIG. 3A).

The inner needle 130 comprises a metal bar (e.g., a stainless steel bar) slidably inserted in the bone cement passage 111 formed in the outer needle 110, and having a sharp needle point 131 on a distal end thereof. The inner needle 130 has an outside diameter, which is substantially the same as the inside diameter of the bone cement passage 111 formed in the outer needle 110. The distal end (cutting edge 112) of the outer needle 110 and the distal end (needle point 131) of the inner needle 130 are combined together to form a needle point 150 (see FIGS. 1 and 3A). The inner needle 130 has a length, which is substantially the same as the sum of the length of the bone cement passage 111 in the outer needle 110 added to the length of the bone cement passage 121 in the outer needle base 120.

The inner needle base 140 comprises a resin-molded member bonded to the proximal end portion of the inner needle 130 (see FIG. 2B). The inner needle base 140 has an internally threaded surface, which is complementary to the externally threaded surface of the outer needle base 120 (see FIG. 3A).

A procedure for injecting bone cement into a bone using the bone cement injection puncture needle 100 thus constituted will be described below by way of example.

First, after a puncture position and a puncture target are determined under image guidance (X-ray fluoroscopy or CT fluoroscopy), the bone cement injection puncture needle 100, including the inner needle 130 mounted therein (see FIGS. 1 and 3A), is inserted into the puncture target in the bone.

After the bone cement injection puncture needle 100 has been inserted into the puncture target, the inner needle 130 is removed from the outer needle 110 (see FIGS. 2A and 3B). At this time, the first side holes 113 are positioned in the bone, while the second side holes 114 remain positioned outside the body of the patient.

Then, a syringe, which is filled with bone cement, is mounted in the opening (insertion port) of the bone cement passage 121 in the outer needle base 120, and then the syringe is operated to inject bone cement through the bone cement passages 121, 111 into the bone. At this time, gases or liquids (e.g., exudate and blood) in the bone enter from the first side holes 113 into the depressurization passages 115, and then flow out of the body of the patient through the second side holes 114. Therefore, almost no pressure buildup occurs inside the bone upon injection of bone cement into the bone.

After a required amount of bone cement has been injected from the syringe into the bone, the syringe is pulled out. Then, the inner needle 130 is inserted into the bone cement passage 121 in the outer needle base 120 as well as into the bone cement passage 111 in the outer needle 110, thereby pushing any remaining bone cement from the bone cement passages 121, 111 into the bone.

Subsequently, if required, the inner needle 130 is removed, and another syringe, which is filled with bone cement, is mounted in the outer needle base 120 again, after which the process of injecting bone cement is repeated.

According to the above procedure, it is possible to inject bone cement into the bone without causing internal pressure buildup in the bone.

### [Second Embodiment]

According to a second embodiment, a bone cement injection puncture needle includes an outer needle, which comprises an outer needle body and an outer tube, and also has first side holes and second side holes formed therein, which can be opened and closed.

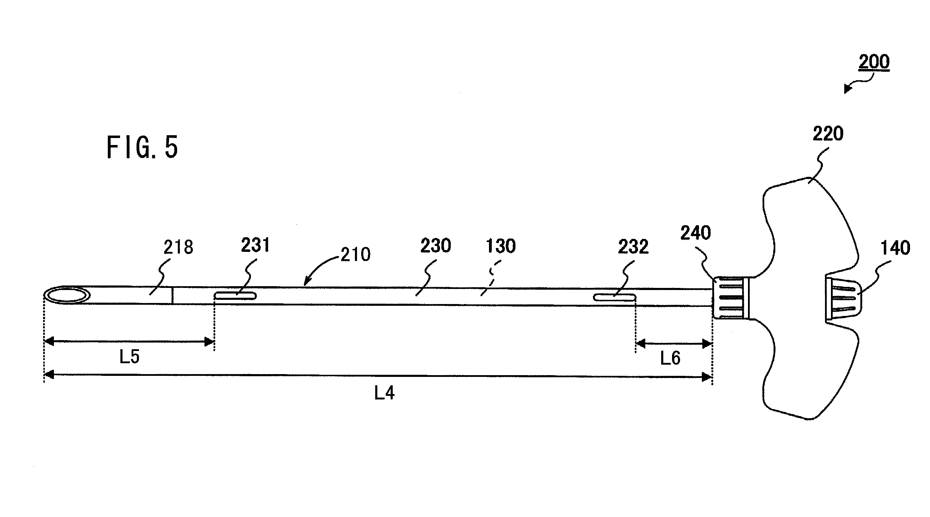

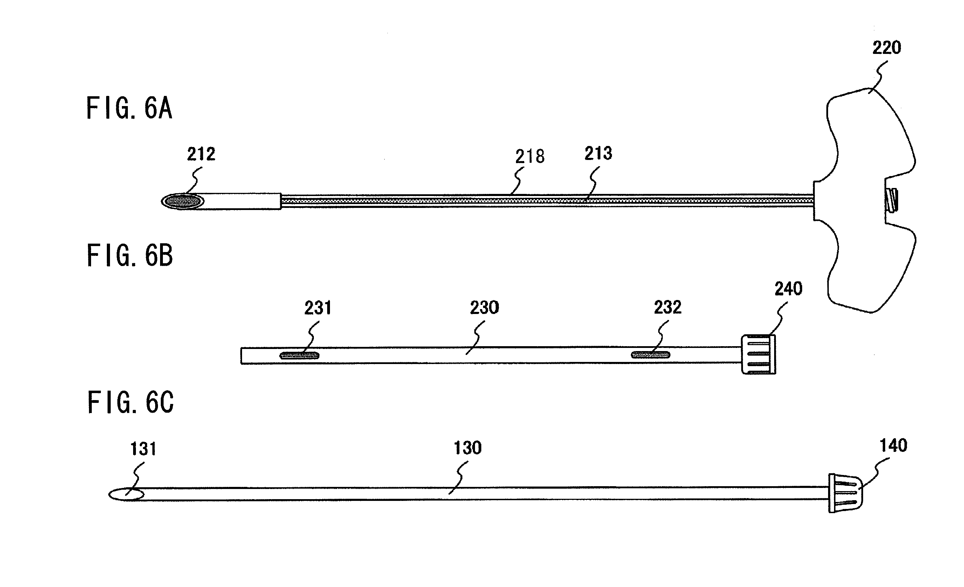

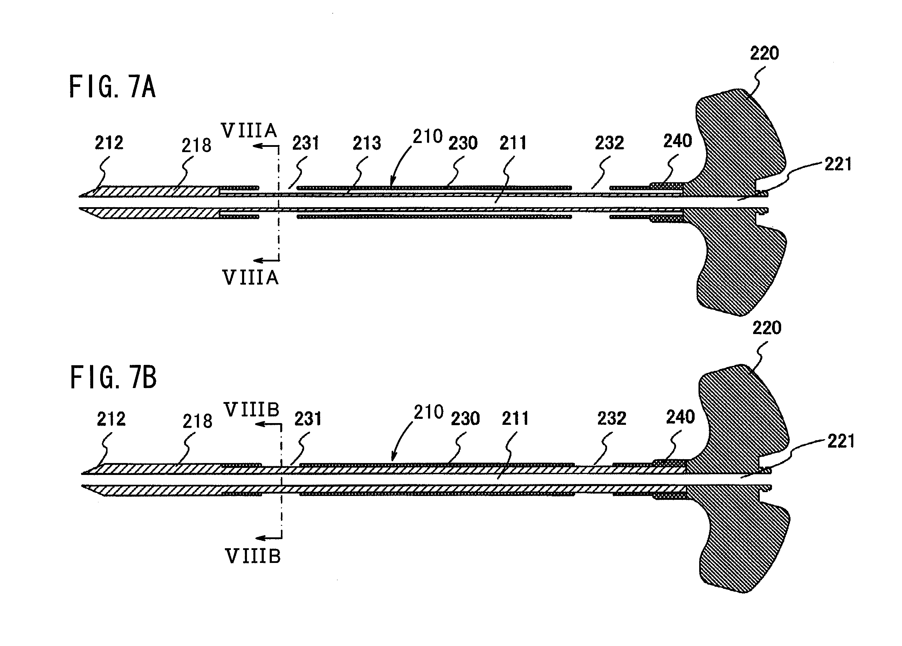

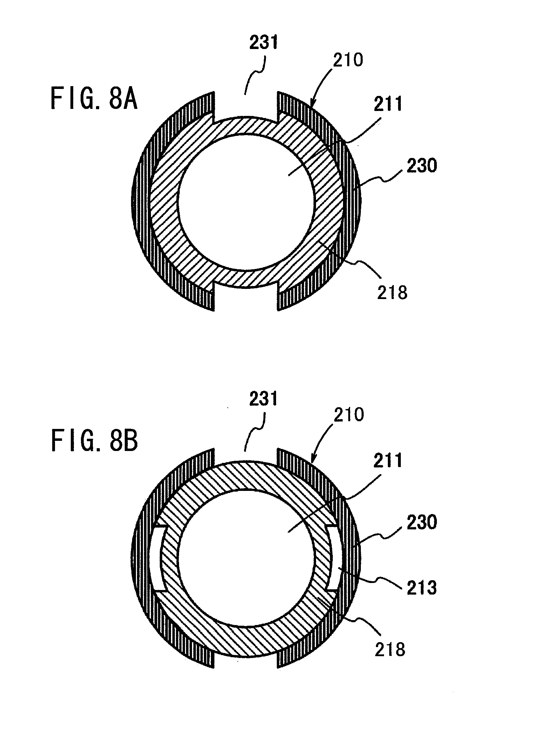

FIGS. 5 to 8 are views showing the structure of the bone cement injection puncture needle according to the second embodiment. FIG. 5 is an overall view of the bone cement injection puncture needle, with an outer needle (an outer needle body and an outer tube) and an inner needle combined together, FIG. 6A is a view of the outer needle body, FIG. 6B is a view of the outer tube, FIG. 6C is a view of the inner needle, FIG. 7A is a cross-sectional view showing the outer needle (the outer needle body and the outer tube) with depressurization passages thereof being open, FIG. 7B is a cross-sectional view showing the outer needle (the outer needle body and the outer tube) with the depressurization passages thereof being closed, FIG. 8A is a cross-sectional view taken along line VIIIA-VIIIA of FIG. 7A, and FIG. 8B is a cross-sectional view taken along line VIIIB-VIIIB of FIG. 7B. In order to more clearly show the internal structure thereof, the ratio of the length and thickness of the needle in the cross-sectional views of FIG. 7 is different from the ratio shown in FIGS. 5 and 6.

As shown in FIGS. 5 to 8, the bone cement injection puncture needle 200 according to the second embodiment comprises an outer needle body 218, an outer needle base 220, an outer tube 230, an outer tube base 240, an inner needle 130, and an inner needle base 140. The outer needle 210 is made up from the outer needle body 218, the outer tube 230, and the outer tube base 240. The inner needle 130 and the inner needle base 140 are identical to the inner needle 130 and the inner needle base 140 of the bone cement injection puncture needle according to the first embodiment, and hence are denoted by the same reference characters.

The outer needle body 218 comprises a metal tube (e.g., a stainless steel tube) having a bone cement passage 211 formed therein, and a sharp cutting edge 212 provided on the distal end thereof. The outer needle body 218 has a length (indicated by "L4" in FIG. 5) in a range from about 10 to 20 cm, and an inside diameter in a range from about 1.8 to 2.4 mm. The outer needle body 218 has a portion, apart from the distal end portion thereof, which is disposed in the outer tube 230. The portion of the outer needle body 218, except for the distal end portion thereof (the portion positioned in the outer tube 230), has an outside diameter, which is smaller than the distal end portion of the outer needle body 218 by an amount equal to the wall thickness of the outer tube 230, so that, when the outer needle body 218 is inserted into the outer tube 230, a step is not formed between the surface of the distal end portion of the outer needle body 218 and the surface of the outer tube 230 (see FIGS. 6A and 7). The portion of the outer needle body 218, except for the distal end portion thereof (the portion positioned in the outer tube 230), has groove-like depressurization passages 213 formed therein. The depressurization passages 213 are not limited to any particular size (width and depth), insofar as gases and liquids can flow therethrough.

The outer needle base 220 comprises a resin-molded member (grip), which is bonded to the proximal end portion of the outer needle body 180 (see FIG. 6A). The outer needle base 220 is not limited to any particular size and shape, but may be of a shape that can be gripped easily by the user (doctor). The outer needle base 220 has a bone cement passage 221 formed therein, which is held in fluid communication with the bone cement passage 211 in the outer needle body 218 (see FIG. 7B). The bone cement passage 221 has an opening, which functions as an insertion port for enabling a syringe to be inserted therein. The outer needle base 220 also has an externally threaded surface, to which the inner needle base 140 is removably fastened (see FIG. 7A).

The outer tube 230 comprises a hollow metal tube (e.g., a stainless steel tube) having first side holes 231 and second side holes 232 formed therein. The first side holes 231 are formed in a side wall of the outer tube 230 near a distal end portion thereof, whereas the second side holes 232 are formed in a side wall of the outer tube 230 near a proximal end portion thereof. When the outer needle body 218 is inserted into the outer tube 230, the distal end portion of the outer needle body 218 is spaced from the first side holes 231 in the outer tube 230 by a distance (indicated by "L5" in FIG. 5) in a range from about 0.5 to 2 cm. The proximal end portion of the outer needle body 218 is spaced from the second side holes 232 in the outer tube 230 by a distance (indicated by "L6" in FIG. 1) in a range from about 0 to 4 cm. The first side holes 231 and the second side holes 232 are not limited to any particular size, insofar as gases and liquids can flow therethrough.

The outer tube base 240 comprises a resin-molded member, which is bonded to the proximal end portion of the outer tube 230 (see FIG. 6B). The outer tube base 240 is not limited to any particular size and shape, insofar as the outer tube base 240 is of a size and shape that allows the user (doctor) to rotate the outer tube 230 easily.

With the bone cement injection puncture needle 200 according to the second embodiment, since the outer needle body 218 with the groove-like depressurization passages 213, and the outer tube 230 with the first side holes 231 and the second side holes 232 are formed as separate members, the outer tube 230 can be rotated with respect to the outer needle body 218. When the outer tube 230 is rotated with respect to the outer needle body 218, until the depressurization passages 213 are brought into fluid communication with the first side holes 231 and the second side holes 232 as shown in FIG. 7A, the depressurization passages 213 are opened with respect to the outer space. When the outer tube 230 is rotated with respect to the outer needle body 218, until the depressurization passages 213 are taken out of fluid communication with the first side holes 231 and the second side holes 232 as shown in FIG. 7B, the depressurization passages 213 are closed with respect to the outer space.

With the bone cement injection puncture needle 200 according to the second embodiment, the single outer needle body 218 has two depressurization passages 213, and the single outer tube 230 has two first side holes 231 and two second side holes 232 formed therein (see FIGS. 7 and 8).

The inner needle 130 and the inner needle base 140 are the same as the inner needle 130 and the inner needle base 140 of the bone cement injection puncture needle according to the first embodiment. The inner needle 130 is slidably inserted in the bone cement passage 211 in the outer needle body 218. The distal end (cutting edge 212) of the outer needle body 218 and the distal end (needle point 131) of the inner needle 130 are combined together to form a needle point (see FIG. 5). The inner needle 130 has a length, which is substantially the same as the sum of the length of the bone cement passage 211 in the outer needle body 218 added to the length of the bone cement passage 221 in the outer needle base 220.

A procedure for injecting bone cement into a bone using the bone cement injection puncture needle 200 thus constituted will be described below by way of example.

First, after a puncture position and a puncture target are determined under image guidance (X-ray fluoroscopy or CT fluoroscopy), the bone cement injection puncture needle 200, including the inner needle 130 mounted therein (see FIG. 5), is inserted into the puncture target in the bone. At this time, the bone cement injection puncture needle 200 is inserted while the first side holes 231 and the second side holes 232 are in a closed state (see FIGS. 7B and 8B).

After the bone cement injection puncture needle 200 has been inserted into the puncture target, the inner needle 130 is removed. The outer tube base 240 and the outer tube 230 are turned in order to open the first side holes 231 and the second side holes 232 (i.e., to bring the first side holes 231 and the second side holes 232 into fluid communication with the depressurization passages 213) (see FIGS. 7A and 8B). At this time, the first side holes 231 are positioned in the bone, while the second side holes 232 are positioned outside the body of the patient.

Then, a syringe, which is filled with bone cement, is mounted in the insertion port of the outer needle base 220, and then the syringe is operated in order to inject bone cement through the bone cement passages 221, 211 and into the bone. At this time, gases or liquids (e.g., exudate and blood) in the bone enter from the first side holes 231 into the depressurization passages 213, and then flow out of the body of the patient through the second side holes 232. Therefore, almost no pressure buildup occurs in the bone upon injection of bone cement into the bone.

After a required amount of bone cement has been injected from the syringe into the bone, the syringe is pulled out. Then, the inner needle 130 is inserted into the bone cement passage 221 in the outer needle base 220 as well as into the bone cement passage 211 in the outer needle body 218, thereby pushing any remaining bone cement from the bone cement passages 221, 211 into the bone.

Subsequently, if required, the inner needle 130 is removed, and another syringe, which is filled with bone cement, is mounted in the outer needle base 220 again, after which more bone cement can be injected.

According to the above procedure, it is possible to inject bone cement into the bone without causing internal pressure buildup in the bone.

Inasmuch as the bone cement injection puncture needle 200 according to the second embodiment can be inserted into the bone while the first side holes 231 and the second side holes 232 are in a closed state, the bone cement injection puncture needle 200 can be inserted into the bone more smoothly than the bone cement injection puncture needle 100 according to the first embodiment.

### [Third Embodiment]

According to a third embodiment, a bone cement injection puncture needle includes an outer needle (an outer needle body and an outer tube), the size of which is different at the distal end portion and the proximal end portion thereof.

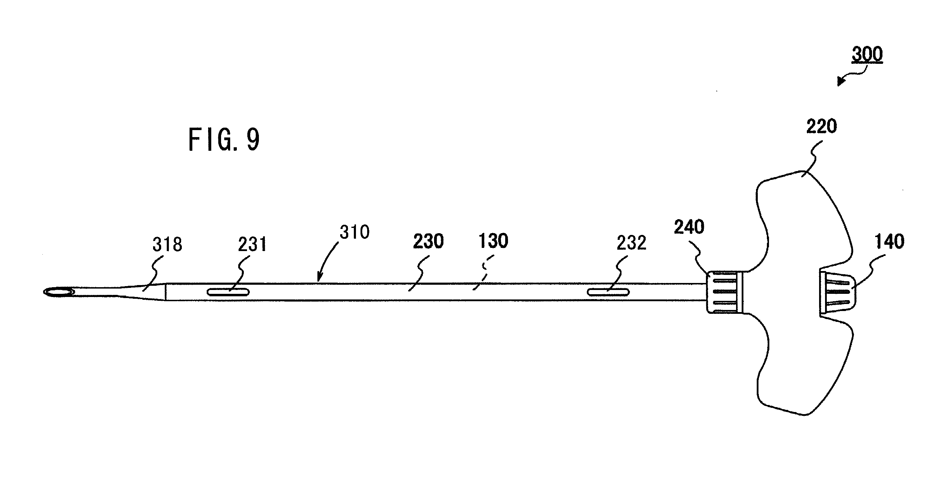

FIG. 9 is a view of the bone cement injection puncture needle according to the third embodiment, i.e., an overall view showing an outer needle (an outer needle body and an outer tube) and an inner needle, which are combined together.

As shown in FIG. 9, the bone cement injection puncture needle 300 according to the third embodiment comprises an outer needle body 318, an outer needle base 220, an outer tube 230, an outer tube base 240, an inner needle 130, and an inner needle base 140. An outer needle 310 includes the outer needle body 318, the outer tube 230, and the outer tube base 240. Components except for the outer needle body 318 are the same as those of the bone cement injection puncture needle according to the second embodiment, and accordingly, such components are denoted by identical reference characters and will not be described in detail below.

The outer needle body 318 comprises a metal tube (e.g., a stainless steel tube) having a bone cement passage formed therein, and a sharp cutting edge on the distal end thereof. The outer needle body 318 has a length in a range from about 10 to 20 cm. The distal end portion of the outer needle body 318 (a portion thereof that is not positioned in the outer tube 230) has an outside diameter of about 2.4 mm (corresponding to 13G). The outer tube 230 has an outside diameter in a range from about 2.7 to 3.0 mm (corresponding to 11 - 12G). The outside diameter of the outer needle body 318 becomes progressively larger from the distal end portion of the outer needle body 318 (having an outside diameter of about 2.4 mm) toward a junction between the outer needle body 318 and the outer tube 230, so that a step is not formed between the surface of the distal end portion of the outer needle body 318 and the surface of the outer tube 230 when the outer needle body 318 is inserted into the outer tube 230 (see FIG. 9).

The bone cement injection puncture needle 300 according to the third embodiment is capable of injecting bone cement into a bone, using the same procedure as the bone cement injection puncture needle 200 according to the second embodiment.

The bone cement injection puncture needle 300 according to the third embodiment can be inserted into the bone more smoothly than the bone cement injection puncture needle 200 according to the second embodiment, because the distal end portion of the outer needle body 318 is thin and free of depressurization passages.

As described above, since the bone cement injection puncture needle according to the present invention has depressurization passages in addition to the cement injection passage therein, the bone cement injection puncture needle is capable of injecting bone cement into a bone without causing internal pressure buildup in the bone. Furthermore, since the bone cement injection puncture needle according to the present invention is capable of injecting bone cement into a bone while gases and liquids are discharged from the bone, the bone cement injection puncture needle can inject bone cement fully into the bone.

### [Fourth Embodiment]

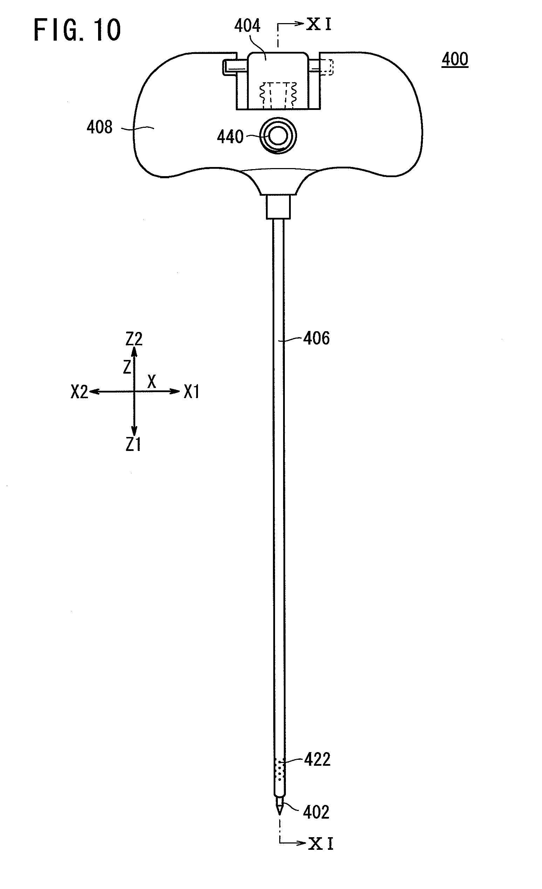

FIG. 10 is an overall view of a bone cement injection puncture needle 400 according to a fourth embodiment of the present invention. As shown in FIG. 10, the bone cement injection puncture needle 400 (hereinafter also referred to as a "puncture needle") comprises a hollow outer needle 406, an outer needle base 408 fixed to a proximal end portion of the outer needle 406, an inner needle 402 slidably inserted in the lumen of the outer needle 406, and an inner needle base 404 fixed to a proximal end portion of the inner needle 402. In FIG. 10, the inner needle 402 is shown as being inserted in the lumen of the outer needle 406.

In the description that follows, axial directions of the inner needle 402 and the outer needle 406 are referred to as Z directions, directions perpendicular to the Z directions are referred to as X directions, and directions perpendicular to both the Z directions and the X directions are referred to as Y directions. In FIG. 1, the X directions are perpendicular to the Z directions and the sheet of the drawing, and the Y directions are perpendicular to the sheet of the drawing. Among the X directions, the rightward direction in FIG. 1 is represented by X1, and the leftward direction is represented by X2. Among the Z directions, the direction toward the distal end portion of the puncture needle 400 is represented by Z1, and the direction toward the proximal end portion of the puncture needle 400 is represented by Z2.

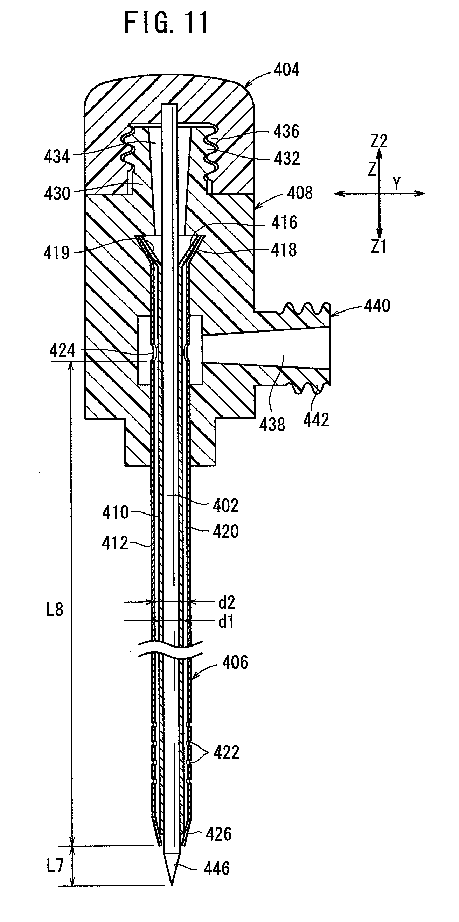

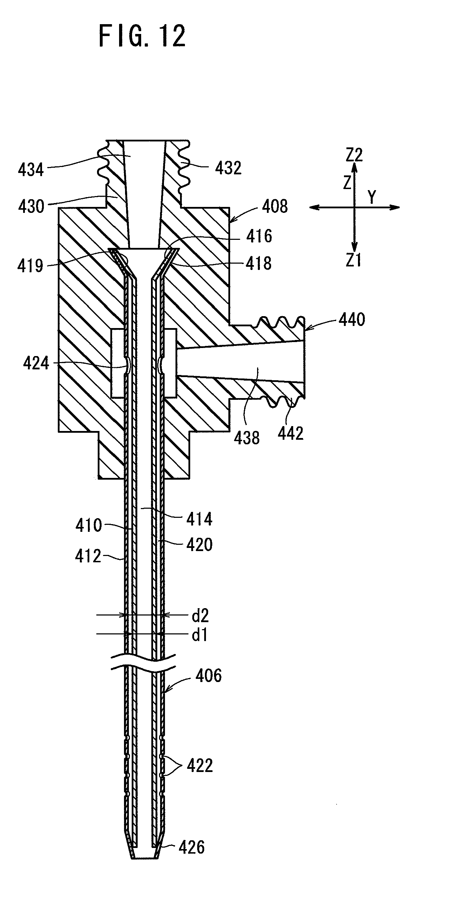

FIG. 11 is a cross-sectional view, partially omitted from illustration, taken along line XI-XI of FIG. 10. FIG. 12 is a cross-sectional view, partially omitted from illustration, of the bone cement injection puncture needle, with the inner needle 402 removed from the outer needle 406. As shown in FIGS. 11 and 12, the outer needle 406 comprises a hollow member with opposite open ends, and includes an inner tube 410, into which the inner needle 402 is inserted, and an outer tube 412 that surrounds the inner tube 410, thereby providing a dual-tube structure. The inner tube 410 and the outer tube 412 may be made of any materials, so long as such materials are strong enough not to become damaged or deformed when the bone cement injection puncture needle is inserted into and pulled out from a bone. Examples of suitable materials are stainless steel, aluminum alloy, copper alloy, or the like.

As shown in FIG. 12, the inner tube 410 has opposite open ends, and also has a bone cement passage 414 formed therein. When the inner needle 402 and the outer needle 406 are combined together, the bone cement passage 414 functions as a hole into which the inner needle 402 can be inserted. When bone cement is injected, the bone cement passage 414 functions as a channel through which the bone cement flows. The inner tube 410 has a length in a range from about 100 to 200 mm. In FIG. 12, the inner tube 410 comprises a hollow cylindrical tube having an inside diameter ranging from 1.8 to 2.4 mm.

The inner tube 410 includes a first flaring portion 416 on a proximal end portion thereof. In FIGS. 11 and 12, the first flaring portion 416 spreads conically toward the proximal end (in the Z2 direction). The angle of the first flaring portion 416 with respect to the axis of the outer needle 406 is set to a value in a range from about 15° to 60°, for example.

The outer tube 412 has opposite open ends, and the inner tube 410 is inserted in the lumen of the outer tube 412. The outer tube 412 has a length in a range from 100 to 200 mm, and is slightly longer than the inner tube 410. The outer tube 412 has an inside diameter d2 greater than the outside diameter d1 of the inner tube 410. An axially extending depressurization passage 420 is formed between the outer tube 412 and the inner tube 410. The inside diameter of the outer tube 412 is in a range from 2.1 to 2.3 mm, for example.

The outer tube 412 has first side holes 422 formed therein near the distal end portion thereof. The first side holes 422 extend between inner and outer spaces of the outer tube 412, and should preferably be provided as a plurality of holes, which are distributed in circumferential and axial directions of the outer needle 406. The number of first side holes 422 is preferably in a range from 4 to 36, and more preferably, in a range from 10 to 26. A preferred layout and dimensions of the first side holes 422 will be described later.

The outer tube 412 has second side holes 424 formed therein near the proximal end portion thereof. The second side holes 424 extend between inner and outer spaces of the outer tube 412. The second side holes 424 (more specifically, regions thereof closer to the distal end (in the Z1 direction)) are spaced from the foremost end of the outer needle 406 by a distance L8, which is set such that when the puncture needle 400 is inserted into a bone, the second side holes 424 are reliably positioned outside the body of the patient. More specifically, the distance L8 is equal to or greater than 80 mm, and more preferably, equal to or greater than 120 mm.

Although the outer tube 412 may have a single second side hole 424, preferably, a plurality of second side holes 424 are provided, which are distributed in circumferential and axial directions. In FIG. 11, two second side holes 424 are spaced from each other in the circumferential direction. The first side holes 422 and the second side holes 424 are held in fluid communication with each other by the depressurization passage 420, which is formed between the outer tube 412 and the inner tube 410.

The outer tube 412 includes a tapered portion 426 on a front end portion thereof, the tapered portion 426 being progressively tapered toward a tip end thereof. The outer tube 412 includes the tapered portion 426, the angle of which with respect to the axis of the outer needle 406 is set to a value in a range from about 1° to 30°, for example. The distal end portion of the inner tube 410 is supported by an inner circumferential surface of the tapered portion 426, thereby closing the distal end of the depressurization passage 420.

The outer tube 412 includes a second flaring portion 418 on a rear end portion thereof. In FIGS. 11 and 12, the second flaring portion 418 spreads conically toward the proximal end (in the Z2 direction). The angle of the second flaring portion 418 with respect to the axis of the outer needle 406 is set approximately the same as the angle of the first flaring portion 416 with respect to the axis of the outer needle 406. The second flaring portion 418 supports the first flaring portion 416. The first flaring portion 416 and the second flaring portion 418 are superposed on each other and are in intimate contact, thereby closing the rear end of the depressurization space.

The outer needle base 408 forms a member, which is coupled to the proximal end portion of the outer needle 406, and which functions as a grip to be gripped by the user of the puncture needle 400. The outer needle base 408 is not limited to any particular material, but may be made of polyvinyl chloride, polyethylene, polypropylene, cyclic polyolefin, polystyrene, poly(4-methylpentene-1), polycarbonate, acrylic resin, acrylonitrile-butadienestyrene copolymer, polyester such as polyethylene terephthalate and polyethylene naphthalate, butadienestyrene copolymer, polyamide (e.g., nylon 6, nylon 6·6, nylon 6·10, nylon 12), or the like.

In FIG. 11, the outer needle base 408 is insert-molded so as to cover and be fixed to the proximal end portion of the outer needle 406. The outer needle base 408 includes a tapered support 419, which is held in abutment against the outer surface of the second flaring portion 418. The second flaring portion 418 is supported by the tapered support 419.

The outer needle base 408 has a passage 434 formed therein, which is held in fluid communication with the bone cement passage 414 in the outer needle 406, and another passage 438 formed therein, which is held in fluid communication with the second side holes 424. The outer needle base 408 also has a main connection port 430 for connection to the inner needle base 404. The main connection port 430 has a lumen that serves as part of the passage 434. The main connection port 430 has an externally threaded outer circumferential surface 432, which is capable of being removably engaged by the inner needle base 404.

As described later, the main connection port 430 also functions as an insertion port, into which a syringe is inserted, which supplies bone cement to the puncture needle 400.

The outer needle base 408 also has an auxiliary connection port 440 on a side surface thereof (a surface facing in the X directions). The auxiliary connection port 440 has a lumen that serves as part of the passage 438. The auxiliary connection port 440 has an externally threaded outer circumferential surface, which is capable of being removably connected to another device or structure.

The inner needle 402 comprises a bar-shaped member, which is inserted into the bone cement passage 414 in the outer needle 406, and which has a sharp cutting edge 446 on the distal end thereof. The inner needle 402 may be made of any material, so long as the material is strong enough so as not to become damaged or deformed when the puncture needle is inserted into and pulled out from a bone. Examples of suitable materials are stainless steel, aluminum alloy, copper alloy, or the like.

The inner needle 402 has an outside diameter, which is substantially the same as the inside diameter of the outer needle 406 (the inside diameter of the inner tube 410). More specifically, the outside diameter of the inner needle 402 may be set to a value that allows the inner needle 402 to be smoothly inserted into the bone cement passage 414, which forms the lumen of the outer needle 406, with essentially no gap created between the outer circumferential surface of the inner needle 402 and the inner circumferential surface of the outer needle 406 (the inner circumferential surface of the inner tube 410).

The inner needle 402 has a length, which is set to a value such that when the inner needle base 404 is connected to the outer needle base 408, the distal end of the inner needle 402 projects slightly from the distal end of the outer needle 406. When the inner needle base 404 is connected to the outer needle base 408, the length by which the inner needle 402 projects from the distal end of the outer needle 406 (i.e., the distance L7 between the distal end of the inner needle 402 and the distal end of the outer needle 406) should preferably be set to a value in a range from 2 to 10 mm. Further, when the inner needle base 404 is connected to the outer needle base 408, the cutting edge 446 (a portion having a cutting face) should be fully exposed from the distal end of the outer needle 406.

The inner needle base 404 comprises a member coupled to the proximal end portion of the inner needle 402. The outside diameter of the inner needle base 404 is greater than the outside diameter of the inner needle 402. More specifically, the outside diameter of the inner needle base 404 is set to a value that allows the user (a medical worker such as a doctor or the like) to hold, push or pull, or turn the inner needle base 404 easily. The inner needle base 404 is not limited to any particular material, but may be made of the same material as the outer needle base 408, e.g., a hard resin such as polycarbonate or the like.

The inner needle base 404 has an internally threaded surface 436, which can be screwed over the externally threaded outer circumferential surface 432 of the main connection port 430 of the outer needle base 408. When the externally threaded outer circumferential surface 432 is screwed into the internally threaded surface 436, the inner needle base 404 becomes connected to the outer needle base 408, thereby keeping the inner needle 402 inserted in the bone cement passage 414 of the outer needle 406.

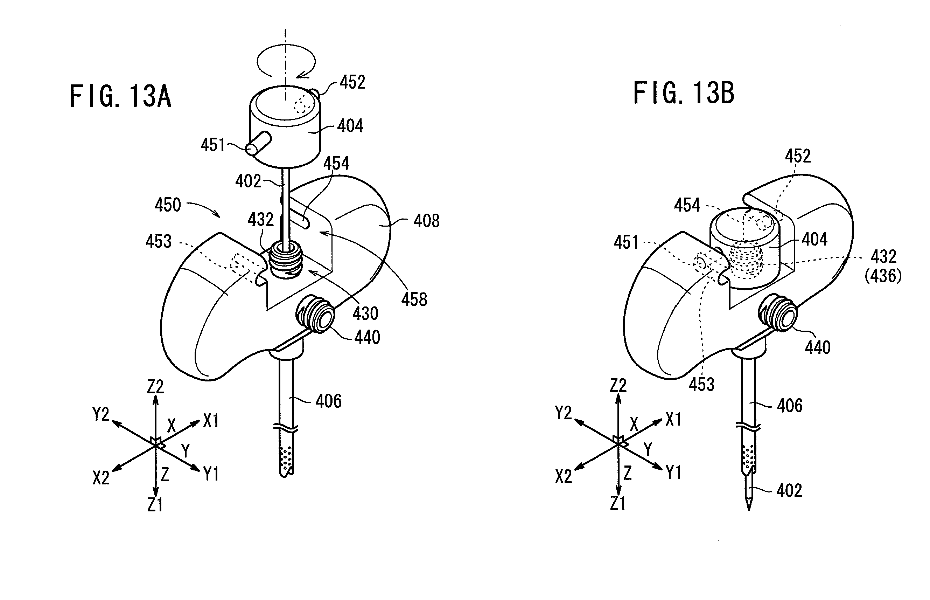

As shown in FIG. 13A, the puncture needle 400 has an assistive support structure 450, for assisting support of the inner needle base 404. The assistive support structure 450 includes a plurality of protrusions 451, 452 that project from the outer circumferential surface of the inner needle base 404, and a plurality of engaging grooves 453, 454, which engage the protrusions 451, 452 when the inner needle base 404 is connected to the outer needle base 408. The protrusions 451, 452 are disposed in symmetrical positions (opposite positions) on the outer circumferential surface of the inner needle base 404, at locations near the upper portion thereof (in the Z2 direction). The engaging grooves 453, 454 extend in a thickness direction (in the Y directions) of the outer needle base 408, at locations near the upper portions of side walls, which form a recess 458 in the outer needle base 408.

Among the two engaging grooves 453, 454, the engaging groove 453, which is disposed in the X2 direction, has an end positioned substantially centrally in the thickness direction of the outer needle base 408, while the other end of the engaging groove 453 opens at an end face of the outer needle base 408 in the Y1 direction. The engaging groove 454, which is disposed in the X1 direction, has an end positioned substantially centrally in the thickness direction of the outer needle base 408, while the other end of the engaging groove 454 opens at an end face of the outer needle base 408 in the Y2 direction. The two engaging grooves 453, 454 thus are disposed in the recess 458, which is formed in the outer needle base 408, in opposite positions in the Y directions. Which of the engaging grooves is disposed in which opposite position in the Y directions is determined based on whether the externally threaded outer circumferential surface 432 is threaded as a right-hand screw or a left-hand screw. With the puncture needle 400 according to the fourth embodiment, since the externally threaded outer circumferential surface 432 (and the internally threaded surface 436) is threaded as a right-hand screw, the two engaging grooves 453, 454 are disposed in positions corresponding to the right-hand screw. If the externally threaded outer circumferential surface 432 (and the internally threaded surface 436) is threaded as a left-hand screw, then the positions of the two engaging grooves 453, 454 in the recess 458, which is formed in the outer needle base 408, are opposite to the positions shown in FIG. 13A with respect to the Y directions.

Since the assistive support structure 450 is constituted as described above, when the inner needle base 404 is screwed over the outer needle base 408 in order to interconnect the inner needle base 404 and the outer needle base 408, the protrusions 451, 452 engage respectively in the engaging grooves 453, 454, as shown in FIG. 13B, due to relative rotation between the inner needle base 404 and the outer needle base 408. Since the protrusions 451, 452 engage respectively within the engaging grooves 453, 454, the inner needle base 404 is supported on the outer needle base 408. Therefore, when a large load is applied from the inner needle base 404 to the outer needle base 408, the load borne by the externally threaded outer circumferential surface 432 and the internally threaded surface 436 is reduced, thereby preventing damage from occurring to the externally threaded outer circumferential surface 432 and the internally threaded surface 436 (i.e., thus preventing the threads thereof from being crushed).

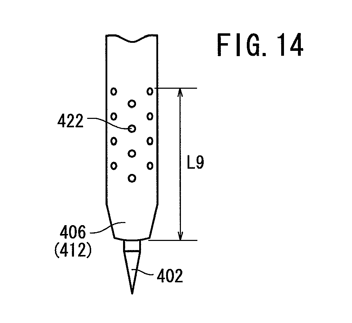

FIG. 14 is an enlarged view, partially omitted from illustration, showing the first side holes 422, which are formed in the outer needle 406, and nearby regions. The distance L9 from the foremost end of the outer needle 406 to first side holes 422 that are positioned most closely to the proximal end (i.e., regions of the first side holes 422, which are positioned most closely to the proximal end) is set to a value such that when the outer needle 406 is inserted into a bone, the first side holes 422, which are positioned most closely to the proximal end, are not positioned outside of the bone. In other words, all of the first side holes 422 are positioned within the bone. More specifically, the distance L9 is equal to or smaller than 20 mm, and more preferably, is equal to or smaller than 15 mm.

If the first side holes 422 are provided as a given number of first side holes 422, then the first side holes 422 may be positioned circumferentially in a zigzag pattern (staggered pattern). For example, the first side holes 422 may be grouped into rows of first side holes 422 along the axis of the outer needle 406, and the first side holes 422 of adjacent rows, which are axially displaced with respect to each other. The first side holes 422 thus arranged are positioned in a well balanced fashion in the outer needle 406, so that the region of the outer needle 406 in which the first side holes 422 are located is prevented from suffering a reduction in mechanical strength.

The first side holes 422 do not need to be of the same size, and may have different sizes. For example, the first side holes 422 may have diameters that become progressively greater toward the distal end of the outer needle 406, so that when a cleaning device is connected to the auxiliary connection port 440 in order to clean the inside of the bone with a cleaning liquid, the amount of cleaning liquid ejected from certain ones of the first side holes 422, which are closer to the proximal end, i.e., the auxiliary connection port 440, will not exceed the amount of cleaning liquid ejected from the first side holes 422 that are closer to the distal end. The first side holes 422 are not required to be circular in shape as shown in FIG. 14, but may be elliptical or polygonal in shape, or may have different mixed shapes.

The first side holes 422 may be set to a size for enabling gases or liquids (e.g., exudate and blood) in the bone to flow smoothly into the outer needle 406. If the first side holes 422 are circular in shape, then the diameters thereof should preferably be in a range from 0.3 to 0.7 mm. If the first side holes 422 are of a shape other than a circular shape, then the dimensions of the narrowest regions thereof should be in a range from 0.3 to 0.7 mm.

If the first side holes 422 are too small, then liquid from within the bone tends to become stuck in the first side holes 422. However, since the size of the first side holes 422 has the above lower limitation, liquid from within the bone is less liable to become stuck in the first side holes 422. If the first side holes 422 are too large, then the outer needle 406 suffers greater resistance upon insertion into the bone, making it less smooth for the user to operate the puncture needle. However, since the size of the first side holes 422 has the above upper limitation, any increase in resistance suffered by the outer needle 406 upon insertion into the bone is reduced.

The bone cement injection puncture needle 400 according to the fourth embodiment is basically constituted as described above. Operations and advantages of the bone cement injection puncture needle 400 will be described below.

For injecting bone cement into a bone using the puncture needle 400, a puncture position and a puncture target are determined under image guidance (X-ray fluoroscopy or CT fluoroscopy). Thereafter, the puncture needle 400, with the inner needle 402 mounted therein, is hit by a hammer until the puncture needle 400 is inserted into the puncture target of the bone. The bone may be a vertebra, for example.

Before the puncture needle 400 is inserted into the patient, a tube for supplying a cleaning liquid may be connected to the main connection port 430, and the cleaning liquid may be supplied through the passage 434 to the bone cement passage 414 in the inner needle 402 in order to clean the bone cement passage 414. Similarly, a tube for supplying a cleaning liquid may be connected to the auxiliary connection port 440, and the cleaning liquid may be supplied through the passage 438 and the second side holes 424 to the depressurization passage 420 disposed between the outer needle 406 and the inner needle 402 in order to clean the depressurization passage 420.

After the puncture needle 400 has been inserted into the puncture target, the inner needle 402 is removed from the outer needle 406. At this time, the first side holes 422 are positioned in the bone and the second side holes 424 are positioned outside the body of the patient.

Then, a syringe filled with bone cement is mounted in the main connection port 430 of the outer needle base 408, and the syringe is operated to inject bone cement through the passage 434 and the bone cement passage 414 into the bone. At this time, gases or liquids (e.g., exudate and blood) in the bone enter from the first side holes 422 into the depressurization passage 420, and then flow out of the body of the patient through the second side holes 424. Therefore, almost no pressure buildup occurs in the bone upon injection of the bone cement into the bone. A suction device may be connected to the auxiliary connection port 440 in order to assist in discharging gases or liquids from the depressurization passage 420.

After a required amount of bone cement has been injected from the syringe into the bone, the syringe is pulled out. Then, the inner needle 402 is inserted into the passage 434 in the outer needle base 408 and into the bone cement passage 414 in the outer needle 406, thereby pushing any remaining bone cement from the passage 434 and the bone cement passage 414 into the bone.

Subsequently, if required, the inner needle 402 is removed, and another syringe, which is filled with bone cement, is mounted once again in the outer needle base 408, after which the process of injecting bone cement is repeated.

With the bone cement injection puncture needle 400 according to the fourth embodiment, as described above, the outer needle 406 has a dual-tube structure with the depressurization passage 420 formed therein. When the puncture needle 400 is inserted into a bone, the first side holes 422 are positioned in the bone, while the second side holes 424 are positioned outside the body of the patient. Gases or liquids in the bone enter from the first side holes 422 into the depressurization passage 420, and then are discharged from the second side holes 424 out of the body of the patient. Since pressure buildup is prevented from developing in the bone upon injection of bone cement into the bone, the bone cement is prevented from leaking out of the bone.

Since there are a plurality of first side holes 422, even if liquid from within the bone sticks to some degree within some of the first side holes 422, the liquid flows from the other first side holes 422 into the outer needle 406. Consequently, pressure buildup is reliably prevented from developing in the bone.

The distance L9 is set to a value equal to or smaller than 20 mm, and preferably equal to or smaller than 15 mm, so that all of the first side holes 422 are positioned in the bone when the puncture needle 400 is inserted into the bone. Therefore, gases and liquids, which flow from within the bone into the outer needle 406, are prevented from leaking into the body of the patient from first side holes 422 that are positioned more closely to the proximal end.

According to the fourth embodiment, since the first flaring portion 416 is supported by the second flaring portion 418, the inner tube 410 and the outer tube 412 are integrally combined together to make up the outer needle 406. Since the second flaring portion 418 is supported by the tapered support 419 of the outer needle base 408, the outer needle 406 is prevented from becoming detached from the outer needle base 408 when the puncture needle 400 is pulled out of the bone. When the outer needle 406 is assembled, the first flaring portion 416 and the second flaring portion 418 are superposed on each other, thereby automatically bringing the inner tube 410 into coaxial alignment with the outer tube 412. Therefore, the inner tube 410 can easily be centered in alignment with the outer tube 412. The inner tube 410 and the outer tube 412 can thus be fabricated easily.

According to the fourth embodiment, the outer tube 412 includes the tapered portion 426 on the front end portion thereof, wherein the tapered portion 426 tapers progressively toward the tip end thereof. The distal end portion of the inner tube 410 is supported by the inner circumferential surface of the tapered portion 426. With this arrangement, when the outer needle 406 is assembled, the inner tube 410 is inserted into the outer tube 412 until the distal end portion of the inner tube 410 abuts against the tapered portion 426 of the outer tube 412. Since the inner tube 410 is automatically brought into coaxial alignment with the outer tube 412, the inner tube 410 can easily be centered in alignment with the outer tube 412. Inasmuch as the distal end portion of the inner tube 410 is supported by the inner circumferential surface of the tapered portion 426 of the outer tube 412, the inner tube 410 and the outer tube 412 do not need to be joined to each other by a joining means such as brazing or the like. The inner tube 410 and the outer tube 412 can thus be fabricated easily.

Furthermore, the puncture needle 400 includes the auxiliary connection port 440. When a cleaning liquid injection tool is connected to the auxiliary connection port 440, the puncture needle 400 can easily and quickly be cleaned. When a suction tool is connected to the auxiliary connection port 440, it is possible to assist in discharging gases or liquids from the depressurization passage 420 of the puncture needle 400.

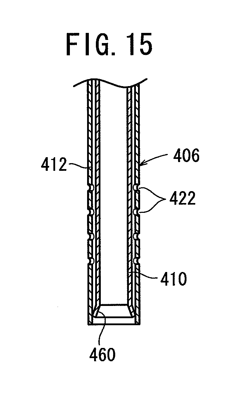

According to the fourth embodiment, the outer tube 412 of the outer needle 406 has the tapered portion 426 located on a front end portion thereof. However, as shown in FIG. 15, the inner tube 410 may have a flaring portion 460 on the front end portion thereof, the flaring portion 460 spreading toward a distal end thereof, and the inner circumferential surface of the front end portion of the outer tube 412 may support an outer circumferential edge of the flaring portion 460.

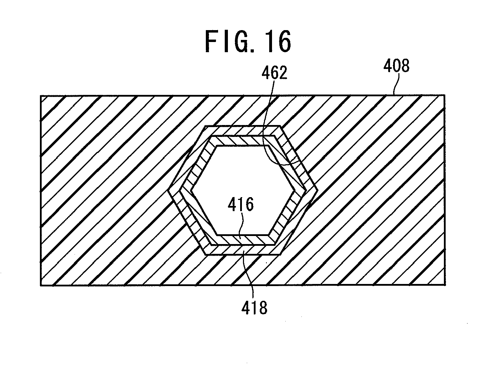

According to the fourth embodiment, both the first flaring portion 416 and the second flaring portion 418 are conical in shape and circular in cross section. However, as shown in FIG. 16, the first flaring portion 416 and the second flaring portion 418 may be polygonal in cross section. The outer needle base 408 may have a tapered support 462, which similarly is polygonal in cross section, and which supports the second flaring portion 418. With this arrangement, the outer needle 406 and the outer needle base 408 are prevented from rotating relatively to each other. Therefore, when the puncture needle 400 is rotated about its axis in order to remove the puncture needle 400 from the bone, since the outer needle base 408 does not rotate with respect to the outer needle 406, the outer needle 406 can be pulled out of the bone easily. In FIG. 16, the first flaring portion 416 and the second flaring portion 418 are hexagonal in cross section. However, the first flaring portion 416 and the second flaring portion 418 may be of a polygonal cross-sectional shape having five or less line segments or seven or more line segments.

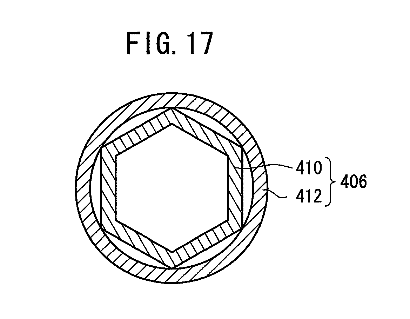

According to the fourth embodiment, the inner tube 410 is of a circular cross-sectional shape. However, as shown in FIG. 17, the inner tube 410 may be partially or wholly polygonal in cross section, such that the inner circumferential surface of the outer tube 412 supports the outer circumferential surface of the inner tube 410. With this arrangement, the outer needle 406 is increased in rigidity. Furthermore, the inner tube 410 may have a plurality of ribs or projections disposed on the outer circumferential surface thereof, instead of a polygonal cross-sectional shape. The ribs or projections may abut against the outer tube 412 so as to be supported by the outer tube 412.

### [Fifth Embodiment]

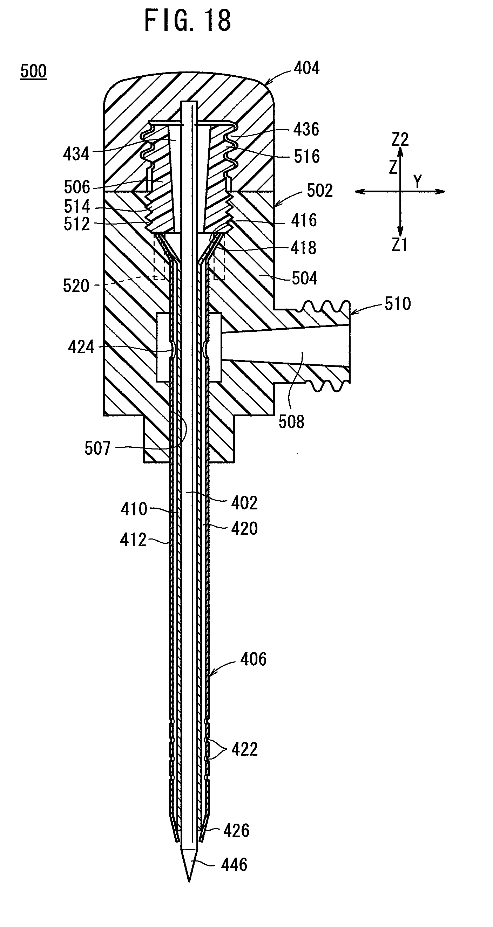

FIG. 18 is a cross-sectional view, partially omitted from illustration, of a bone cement injection puncture needle 500 (hereinafter referred to as a "puncture needle") according to a fifth embodiment of the present invention. Parts of the puncture needle 500 according to the fifth embodiment, which have functions and advantages identical to those of the puncture needle 400 according to the fourth embodiment, are denoted by identical reference characters, and such features will not be described in detail below.

The puncture needle 500 according to the fifth embodiment differs from the puncture needle 400 according to the fourth embodiment as to the structure of the outer needle base 408. The outer needle base 408 according to the fourth embodiment comprises the main connection port 430, and another portion that surrounds the outer needle base 408, which are integrally insert-molded together. The outer needle base 408 according to the fifth embodiment comprises a main body 502 that surrounds the outer needle base 408, and a stopper member 506 engaged with and fixed to the main body 502.

The main body 502 and the stopper member 506 may be made of the same material as the outer needle base 408 according to the fourth embodiment. The main body 502 and the stopper member 506 may also be made of different materials, respectively.

The main body 502 has a passage 508 that is held in fluid communication with the second side holes 424, an outer needle insertion hole 507 through which the outer needle 406 is inserted, and an auxiliary connection port 510. The passage 508 and the auxiliary connection port 510 have structural details and functions, which are identical to those of the passage 438 and the auxiliary connection port 440 (see FIG. 11) according to the fourth embodiment.

The stopper member 506 serves to sandwich and secure the first flaring portion 416 and the second flaring portion 418 of the outer needle 406 between the stopper member 506 and the main body 502. The stopper member 506 has a first externally threaded surface 514, which is held in screw engagement with an internally threaded surface 512 of the main body 502. The stopper member 506 also has a second externally threaded surface 516, which is held in screw engagement with the internally threaded surface 436 of the inner needle base 404.

As shown in FIG. 18, the puncture needle 500 has stop pins 520 extending through the first flaring portion 416 and the second flaring portion 418, and which are inserted into the main body 502. The stop pins 520 prevent the first flaring portion 416 and the second flaring portion 418 from rotating with respect to the main body 502. Other structural details of the puncture needle 500 are identical to those of the puncture needle 400 according to the fourth embodiment.

When the puncture needle 500 according to the fifth embodiment is inserted into a bone and bone cement is injected into the bone, gases or liquids in the bone can flow from the first side holes 422 into the depressurization passage 420, and then flow from the second side holes 424 out of the body of the patient. Therefore, as with the fourth embodiment, pressure buildup is prevented from developing in the bone upon injection of bone cement into the bone.

Parts of the fifth embodiment that are common with those of the fourth embodiment operate in an identical or similar manner, and offer identical or similar advantages to those of the fourth embodiment.

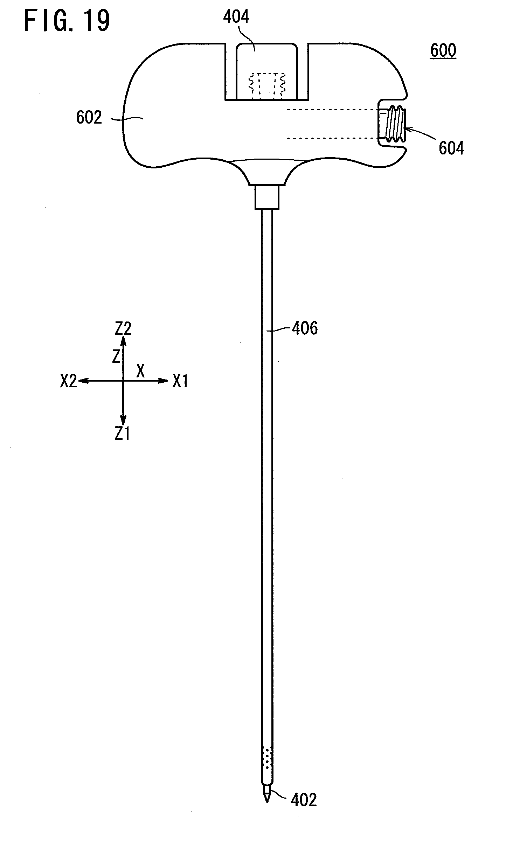

According to the fourth and fifth embodiments, the auxiliary connection ports 440, 510 are disposed on the side surface of the outer needle base 408 (the surface facing in the Y direction). However, as shown in FIG. 19, a bone cement injection puncture needle 600 may have an auxiliary connection port 604 disposed on one of left and right ends (ends in the X directions) of an outer needle base 602.

In percutaneous vertebroplasty, when a plurality of bone cement injection puncture needles are used, the bone cement injection puncture needles may be inserted into the body of a patient in directions that maintain the outer needle bases parallel to each other. With the bone cement injection puncture needle 600 shown in FIG. 19, the auxiliary connection port 604 is disposed on a longitudinal end of the outer needle base 602. Auxiliary connection ports 604 of a plurality of puncture needles 600, which are used adjacent to each other, do not interfere with each other and thus allow the user to operate the puncture needles smoothly.

Although preferred embodiments of the present invention have been described above, it should be understood that the present invention is not limited to the aforementioned embodiments. Various changes and modifications may be made to such embodiments without departing from the scope of the invention as set forth in the appended claims.

## Claims

1. A bone cement injection puncture needle (100, 200, 300) comprising:
a hollow outer needle (110, 210, 310) having a cutting edge (112, 212) on a distal end thereof;
an outer needle base (120, 220) fixed to a proximal end portion of the outer needle (110, 210);
an inner needle (130) having a needle point (131) on a distal end thereof and slidably inserted in a lumen of the outer needle (110, 210); and
an inner needle base (140) fixed to a proximal end portion of the inner needle (130),
wherein the outer needle (110, 210, 310) includes a first side hole (113, 231) positioned near a distal end portion thereof, a second side hole (114, 232) positioned near a proximal end portion thereof, and a depressurization passage (115, 213) interconnecting the first side hole (113, 231) and the second side hole (114, 232).

2. The bone cement injection puncture needle (200, 300) according to claim 1, wherein the outer needle (210, 310) includes a hollow outer tube (230) having the first side hole (231) and the second side hole (232), and a hollow outer needle body (218, 318) rotatably inserted in a lumen of the outer tube (230) and having a groove formed in an outer surface thereof and a sharp cutting edge (212) on a distal end thereof;
the inner needle is slidably inserted in a lumen of the outer needle body (218, 318); and
when the outer tube (230) is rotated with respect to the outer needle body (218, 318), the first side hole (231) and the second side hole (232) are brought into fluid communication with the groove to thereby provide the depressurization passage (213).

3. The bone cement injection puncture needle (200, 300) according to claim 2, further comprising:
an outer tube base (240) fixed to a proximal end portion of the outer tube (230).

4. The bone cement injection puncture needle (100, 200, 300) according to claim 1, wherein the outer needle (110, 210, 310) has a length in a range from 10 to 20 cm; and
the first side hole (113, 231) and the distal end of the outer needle (110, 210, 310) are spaced from each other by a distance in a range from 0.5 to 2 cm, and the second side hole (114, 232) and the proximal end of the outer needle (110, 210, 310) are spaced from each other by a distance in a range from 0 to 4 cm.

5. The bone cement injection puncture needle (100, 200, 300) according to claim 1, wherein the outer needle (110, 210, 310) has an inside diameter in a range from 1.6 to 3.8 mm.

6. A bone cement injection puncture needle (400, 500, 600) comprising:
a hollow outer needle (406);
an outer needle base (408, 502, 602) fixed to a proximal end portion of the outer needle (406);
an inner needle (402) having a needle point (446) on a distal end thereof and slidably inserted in a lumen of the outer needle (406); and
an inner needle base (404) fixed to a proximal end portion of the inner needle (402),
wherein the outer needle (406) includes an inner tube (410) with the inner needle (402) inserted therein, and an outer tube (412) surrounding the inner tube (410).

7. The bone cement injection puncture needle (400, 500, 600) according to claim 6, wherein the outer tube (412) includes a first side hole (422) positioned near a distal end portion thereof, and a second side hole (424) positioned near a proximal end portion thereof, the first side hole (422) and the second side hole (424) being held in fluid communication with each other through a depressurization passage (420) formed between the inner tube (410) and the outer tube (412).

8. The bone cement injection puncture needle (400, 500, 600) according to claim 6, wherein the inner tube (410) has a first flaring portion (416) on a proximal end portion thereof;
the outer tube (412) has a second flaring portion (418) on a proximal end portion thereof;
the first flaring portion (416) is supported by the second flaring portion (418); and
the outer needle base (408, 502, 602) has a tapered support (419) held in abutment against an outer surface of the second flaring portion (418).

9. The bone cement injection puncture needle (400, 500, 600) according to claim 8, wherein the first flaring portion (416) and the second flaring portion (418) are polygonal in cross section.

10. The bone cement injection puncture needle (400, 500, 600) according to claim 6, wherein the outer tube (412) has a tapered portion (426) on a distal end portion thereof, the tapered portion (426) being progressively tapered toward a tip end thereof; and
the inner tube (410) has a distal end portion supported by an inner circumferential surface of the tapered portion (426).

11. The bone cement injection puncture needle (400, 500, 600) according to claim 6, further comprising:
an auxiliary connection port (440, 510, 604) fixed to the outer needle (406) and having a passage held in fluid communication with the second side hole (424).

12. The bone cement injection puncture needle (400, 500, 600) according to claim 11, wherein the auxiliary connection port (440, 510, 604) is formed integrally with the outer needle base (408, 502, 602).

13. The bone cement injection puncture needle (400, 500, 600) according to claim 7, wherein the first side hole (422) comprises a plurality of side holes distributed in circumferential and axial directions of the outer needle (406).

14. The bone cement injection puncture needle (400, 500, 600) according to claim 13, wherein the outer needle (406) has a foremost end spaced from certain ones of the first side holes (422), which are positioned most closely to the proximal end of the outer needle (406) by a distance equal to or smaller than 20 mm.
